# EUROPEAN PATENT SPECIFICATION

(11) **EP 2 772 216 B1**
(45) Date of publication and mention of the grant of the patent: **08.11.2017**
(21) Application number: 14169914.0
(22) Date of filing: 05.08.2011
(51) Int. Cl.: A61B 17/70, A61B 90/00

(54) **Locking device for locking a rod-shaped element in a receiving part of a bone anchor and bone anchor with such a locking device**
Feststellvorrichtung zum Feststellen eines stabförmigen Elements im Aufnahmeteil eines Knochenankers und Knochenanker mit solch einer Feststellvorrichtung
Dispositif de verrouillage pour verrouiller un élément en forme de tige dans une partie réceptrice d'un ancrage d'os et ancrage d'os doté d'un tel dispositif de verrouillage

(43) Date of publication of application: 03.09.2014
(62) Divisional of application: 11176723.2
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE)
(74) Representative: Prüfer & Partner mbB Patentanwälte · Rechtsanwälte

(56) References cited:
- EP-A1- 2 160 988
- WO-A1-2007/075454
- DE-C1- 10 157 969
- DE-U1-202007 012 643
- US-A1- 2010 262 195

## Description

The invention relates to a locking device for securing a rod-shaped element in the receiving part of a bone anchor and to a bone anchor with such a locking device. The locking device is composed of a first locking element and a second locking element that is contained in the first locking element and that is protected against backing out or loss. The invention is particularly applicable to a polyaxial bone screw, wherein the head of the screw element and the rod can be fixed independently.

A polyaxial bone screw wherein the head of the screw element and the rod can be fixed independently is known, for example, from US 7,223,268 B2. The locking device described therein has a first locking element cooperating with a thread provided at the inner wall of the receiving part and a second locking element in the form of a set screw that is contained in the first locking element.

Document DE 20 2007 012 643 U1 discloses a locking device for securing a rod-shaped element in a receiving part of a bone anchor. The locking device comprises a first and a second locking member. The first locking member is provided with an inner thread and the second locking member is provided with an outer thread cooperating with the inner thread of the first locking member. The first and second locking member respectively have two ends, the ends of the second locking member being located outside of a bore of the first locking member when the position of the second locking member is defined by a stop.

A nested fastener and set screw combination for securing a spinal fixation rod to a bone screw, is described in US 7,204,838 B2. The fastener base has a central threaded bore to receive a threaded set screw. The fastener base is provided with a radially inwardly extending abutment shoulder to engage and abut the set screw, prohibiting advancement of the set screw out of a top of the fastener and allowing for a simultaneous removal of the set screw and the fastener.

It is the object of the invention to provide an improved locking device and a bone anchor with such a locking device that exhibits enhanced safety during manipulation in the course of a surgery.

The object is solved by a locking device according to claim 1 and by a bone anchor according to claim 13. Further developments are given in the dependent claims.

The second locking element is contained in the first locking element in such a way that the second locking element is not separable from the first locking element during use. Therefore, if an operating error with regard to an instrument for inserting the locking device happens or when the second locking element is loosened again during further adjustments of the position of the rod, the second locking element cannot escape out of the first locking element and therefore cannot get lost.

The locking device can be delivered in a pre-assembled manner. Because the second locking element is contained in the first locking element in a manner protected against backing out or loss, the handling during surgery is facilitated and security is enhanced.

Although the locking device is particularly applicable to a polyaxial bone anchor that is configured to allow an independent head and rod fixation, it can also be used with monoaxial bone anchors or with bone plates having bone screws with variable angle placement.

Further features and advantages of the invention will become apparent from the description of embodiments by means of the accompanying drawings. In the drawings:
- Fig. 1:: shows a perspective exploded view of an embodiment of a polyaxial bone screw with a first embodiment of the locking device.
- Fig. 2:: shows a perspective view of the polyaxial bone screw with the locking device of Fig. 1 in an assembled state.
- Fig. 3:: shows a cross-sectional view of the first locking element of the locking device according to the first embodiment, the section being taken in a plane containing the central bore axis.
- Fig. 4:: shows a perspective view from the top of the first locking element according to Fig. 3.
- Fig. 5:: shows a perspective view from the bottom of the first locking element of Fig. 3.
- Fig. 6:: shows a cross-sectional view of the second locking element of the locking device according to the first embodiment, the section taken in a plane containing the central bore axis.
- Fig. 7:: shows a perspective view from the top of the second locking element of Fig. 6.
- Fig. 8:: shows a perspective view from the bottom of the second locking element of Fig. 6.
- Figs. 9 to 11:: show steps of assembling the locking device according to the first embodiment in cross-sectional views, the section being taken in a plane containing the central bore axis.
- Fig. 12a:: shows a cross-sectional view of the locking device of the first embodiment with a portion of a tool in a first position, the section being taken in a plane containing the central bore axis of the locking device.
- Fig. 12b:: shows an enlarged portion of Fig. 12a.
- Fig. 13a:: shows a cross-sectional view of the first embodiment of the locking device with a portion of a tool in a second position, the section being taken in a plane containing the central bore axis of the locking device.
- Fig. 13b:: shows an enlarged portion of Fig. 13a.
- Fig. 14:: shows a cross-sectional view of the polyaxial bone screw of Fig. 1 with the locking device according to the first embodiment and a portion of the tool in an assembled state, the section being taken in a plane containing the central bore axis of the locking device and the screw axis.
- Fig. 15:: shows a cross-sectional view of the polyaxial bone screw with the locking device according to the first embodiment without the tool.
- Fig. 16:: shows a perspective exploded view of the locking device according to a second embodiment.
- Figs. 17 to 19:: show steps of assembling the locking device according to the second embodiment in a perspective view from the top.
- Fig. 20:: shows a perspective view from the top of the first locking element of the locking device according to the second embodiment.
- Fig. 21:: shows a cross-sectional view of the first locking element according to Fig. 20, the section taken in a plane containing the central bore axis.
- Fig. 22:: shows a cross-sectional view along line A-A of the first locking element shown in Fig. 21.
- Fig. 23:: shows a cross-sectional view of the second locking element of the locking device according to the second embodiment, the section being taken in a plane containing the central bore axis.
- Fig. 24:: shows a top view of the second locking element shown in Fig. 23.
- Fig. 25:: shows a perspective view of the second locking element according to Fig. 23.
- Fig. 26:: shows a cross-sectional view of the locking device according to the second embodiment with the second locking element in a first position, the section being taken in a plane containing the central bore axis.
- Fig. 27:: shows a cross-sectional view of the second locking device shown in Fig. 26 along line B-B in Fig. 26.
- Fig. 28:: shows a cross-sectional view of the locking device according to the second embodiment with the second locking element in a second position, the section being taken in a plane containing the central bore axis.
- Fig. 29:: shows a cross-sectional view of the locking device of Fig. 28 along line D-D in Fig. 28.
- Fig. 30:: shows a cross-sectional view of the second locking element according to a modification.
- Fig. 31:: shows a perspective view from the top of the second locking element according to the modification.
- Fig. 32:: shows a top view of the second locking element according to the modification.
- Fig. 33:: shows a perspective view from the bottom of the second locking element according to the modification.
- Fig. 34a:: shows a cross-sectional view of the locking device according to the modification.
- Fig. 34b:: shows an enlarged portion of Fig. 34a.
- Fig. 35a:: shows a cross-sectional view of the locking device according to the modification.
- Fig. 35b:: shows an enlarged portion of Fig. 35a.

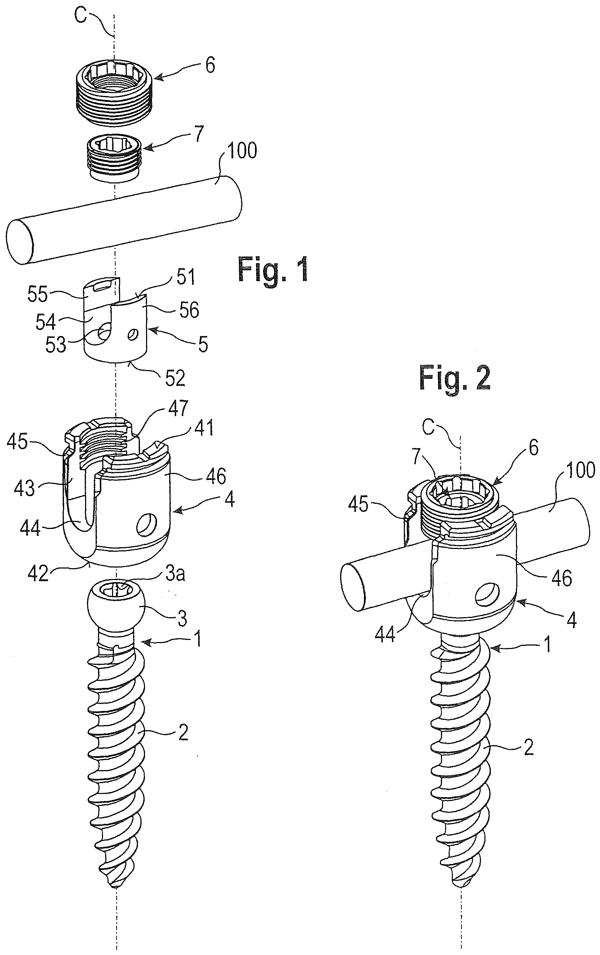

An embodiment of a polyaxial bone screw is shown in Figs. 1 and 2. The polyaxial bone screw comprises a screw element 1 with a threaded shank 2 and a head 3. The head 3 has the shape of a segment of a sphere and comprises at its free end a recess 3a for engagement with a screw driver. The screw element 3 is pivotably held in a receiving part 4. The receiving part 4 is substantially cylindrical with a first end 41 and a second end 42 and a seat for the head 3 near the second end 42, which will be described later. A coaxial bore 43 extends from the first end 41 to the direction of the second 42. Furthermore, a U-shaped recess 44 is provided at the first end 41 by means of which two free legs 45, 46 are formed. An internal thread 47 is provided at the inner wall of said legs 45, 46. The U-shaped recess 44 and the legs 45, 46 form a channel for receiving a rod 100. A pressure element 5 is provided that is configured to exert pressure onto the head for locking the head 3 in a particular angular orientation of the screw element 1 in relation to the receiving part 4. The pressure element 5 is substantially cylindrical with a first end 51 and an opposite second end 52, a coaxial bore 53 and a U-shaped recess 54 extending from the first end 51 into the direction of the second end 52. By means of the U-shaped recess 54 two free legs 55, 56 are formed that also form a channel for receiving the rod 100. The height of the legs 55, 56 in the embodiment shown is greater than the diameter of the rod 100, so that the legs 55, 56 extend above the rod 100 when the rod is inserted.

The bone anchor further includes a locking device comprising a first locking element 6 and a second locking element 7. In the assembled state, the second locking element 7 is nested within the first locking element 6 and is movable between a first position and a second position in an axial direction along a central axis C.

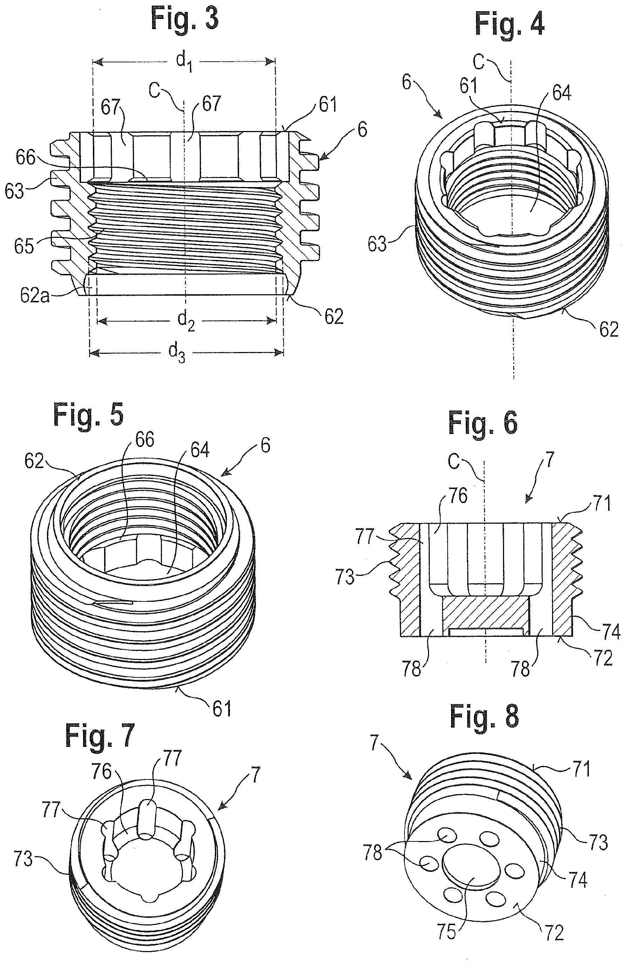

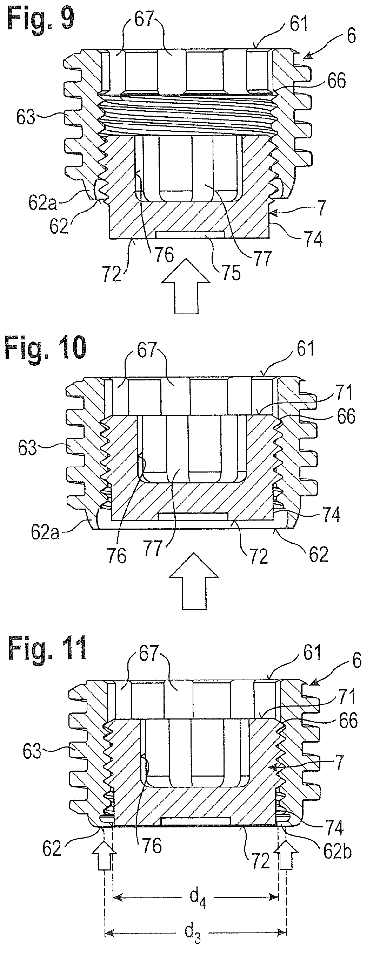

The locking device according to the first embodiment will now be described in more detail with reference to Figs. 3 to 11. Figs. 3 to 5 show the first locking element 6 before assembly with the second locking element 7. The first locking element 6 is substantially cylindrical, and has a first end 61 and a second end 62. At least a portion of the outer surface of the first locking element 6 comprises an external thread 63 that is configured to cooperate with the internal thread 47 provided on the legs of the receiving part 4. The first locking element 6 is provided with a coaxial bore 64 with a bore axis C and with a portion 65 comprising an internal thread. The portion 65 is adjacent or near the second end and extends up to a distance from the first end 61. The thread form of the external thread 63 is preferably a thread form that prevents splaying of the legs 45, 46 of the receiving part, such as a flat thread (as shown) or a negative angle thread with a negative angle of the load-bearing surface. The internal thread can have any thread form like a metric thread as shown for example in Figs. 9 to 11 but can also have a flat thread.

The first locking element 6 further comprises at the second end 62 a threadless concavely rounded hollow cylindrical portion 62a that is, after insertion of the second locking element, bent inwards as described below. Adjacent the first end 61 the coaxial bore has an inner diameter d₁ that is the same or smaller than an inner diameter d₂ measured between the crests of the thread of the internally threaded portion 65 but smaller than a diameter d₃ between the thread roots of the internally threaded portion 65. By means of this, a stop in form of an abutment 66 is provided for the advancement of the second locking element 7 to the direction of the first end 61 of the first locking element 6. Adjacent the first end 61, an engagement structure 67 for engagement with a tool is provided. The engagement structure 67 can be a plurality of coaxial semi-spherical grooves.

The second locking element 7 is substantially cylindrical and has a first end 71 and an opposite second end 72. Adjacent the first end 71, a portion 73 with an external thread is provided that is configured to cooperate with the internally threaded portion 65 of the first locking element 6. Adjacent the second end 72, a threadless portion 74 is provided. The outer surface of the portion 74 is substantially smooth. An outer diameter of the threadless portion 74 is smaller than an outer diameter of the threaded portion 73. The second locking element 7 further comprises a coaxial bore, i.e. a recess 75 and a coaxial recess 76 adjacent the first end 71 that has an inner diameter that is greater than that of the recess 75. Furthermore, adjacent the first end 71, an engagement structure in the form of a plurality of coaxial longitudinal grooves 77 is provided that serve for engagement with an insertion and drive tool. The coaxial longitudinal grooves 77 open into the face of the second end 72 forming circular holes 78 there. The holes 78 can serve for engagement with a tool to mount the second locking element into the first locking element from the second end 62.

The steps of assembling the first and the second locking element will be explained referring to Figs. 9 to 11. In a first step, the second locking element 7 is introduced into the first locking element 6 from the second end 62 of the first locking element 6. Then, as shown in Fig. 10, the second locking element 7 is advanced by screwing it in the direction to the first end 61 until its first end 71 abuts against the abutment 66 provided at the coaxial bore of the first locking element 6. The length of the second locking element 7 in an axial direction is such that when the second locking element 7 assumes a first position, wherein its first end 71 abuts against the abutment 66, the second end 72 is located within the concavely rounded hollow cylindrical portion 62a. Thereafter, as shown in Fig. 11, the concavely rounded hollow cylindrical portion 62a is bent inwards until it nearly touches the threadless portion 74 of the second locking element 7. By this operation, a second stop in form of an annular abutment 62b is formed. The inner diameter d₄ of the annular abutment 62b is smaller than the inner diameter d₃ between the roots of the threaded portion 65 of the first locking element 6 but greater than an outer diameter of the threadless portion 74 of the second locking element 7.

The parts of the polyaxial bone screw and the locking device are all made from a biocompatible material. Such a biocompatible material can be titanium, stainless steel, biocompatible alloys, such as titanium nickel alloys, for example Nitinol, or can be a biocompatible plastic material, such as PEEK (polyetheretherketone). The components can be made all of the same material or of different materials.

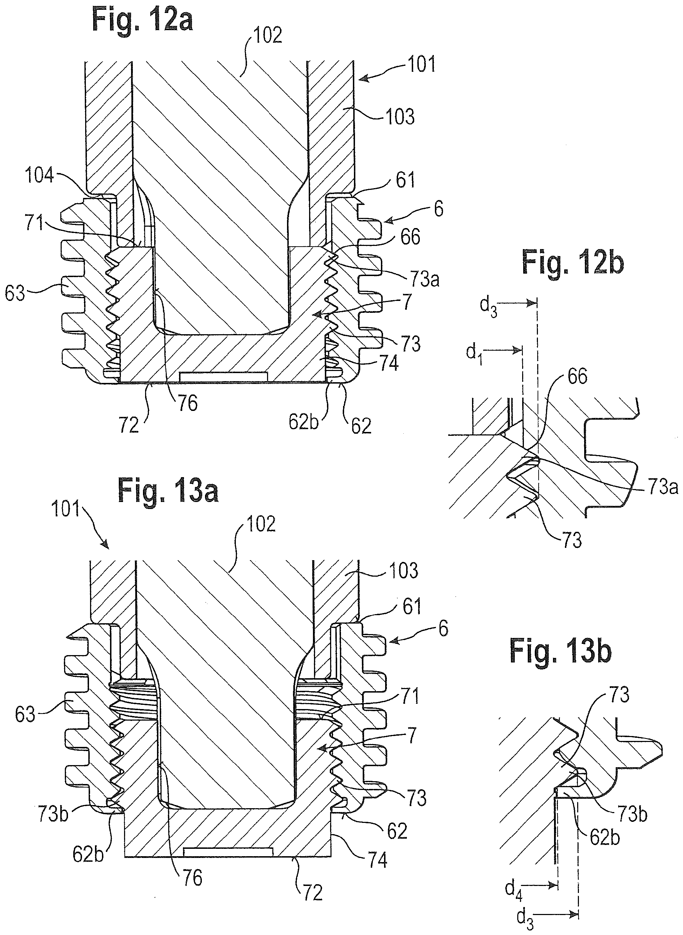

The function of the locking device is shown in Figs. 12a-13b. In Fig. 12a a tool 101 is shown that comprises an inner drive portion 102 and an outer drive portion 103. The inner drive portion engages the recess 76 and the engagement structure 77 of the second locking element. The outer drive portion 103 engages the engagement structure 67 in the first locking element 6. The inner drive portion 102 and the outer drive portion 103 can be rotated independently. By means of the inner drive portion 102, the second locking element 7 is rotated so that it is advanced towards the first end 61 of the first locking element 6 until, as shown in Figs. 12a and 12b, the first thread turn 73a adjacent the first end 71 of the second locking element abuts against the abutment 66 formed by the inner wall of the coaxial bore 64. Because the inner diameter d₁ of the inner wall of the coaxial bore 64 is smaller than the inner diameter d₃ at the root of the internal thread 63 of the first locking element, the second locking element 7 cannot advance further towards the first end and is at the first end position. When the second locking element 7 is in the first end position, a gap 104 is present between the first end 61 of the first locking element and the outer drive portion 103.

By rotating the first drive portion 102 and by moving down the second drive portion 103 so that it abuts against the first end 61 of the first locking element 6, the second locking element 7 advances in the direction of the second end 62 of the first locking element 6. The advancement stops, when a last thread turn 73b that is opposite to the first thread turn 73a abuts against the second stop in form of the annular abutment 62b. Because the inner diameter d₄ of the annular abutment 62b is smaller than the inner diameter d₃ between the roots of the threaded portion 63 of the first locking element 6, the second locking element 7 cannot be screwed further towards the second end 62 and, therefore, cannot escape. When the second locking element 7 is in the second end position, the threadless portion 74 projects out of the second end 62 of the first locking element.

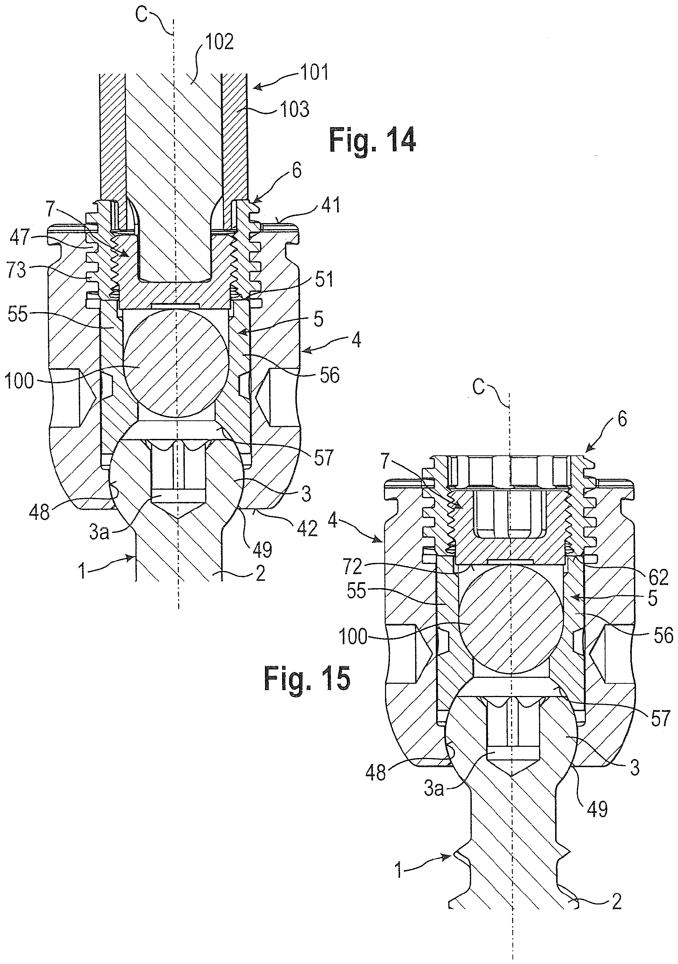

The use of the locking device according to the first embodiment is shown in Figs. 14 and 15. Fig. 14 shows the polyaxial bone screw according to Figs. 1 and 2 in an assembled state. To allow pivoting before fixation, the receiving part 4 has a seat portion 48, wherein the screw head 3 is held. The seat portion can be spherically-shaped. The screw head and the threaded shank 2 extend through an opening 49 provided at the second end 42 of the receiving part 4. The pressure element 5 is arranged on top of the head 3 and encompasses partially the head 3 with a spherically-shaped recess 57. The pressure element 5 and the receiving part 4 are aligned such that the U-shaped recesses 44, 54 are in alignment to allow the insertion of the rod 100. When the rod 100 rests in the U-shaped recess 54 of the receiving part 5, the legs 55, 56 extend above the rod 100.

The locking device is inserted into the receiving part in a pre-assembled manner in which the second locking element 7 is contained in the first locking element 6. With the outer drive portion 103, the first locking element 6 is screwed into the internal thread 47 of the receiving part 4 until its second end 62 abuts against the upper free surface of the legs 55, 56 of the pressure element. By further advancement of the first locking element 6, the pressure element 5 exerts pressure onto the head 3 until the head 3 is finally locked in a desired angular position of the threaded shank 2 with respect to the central axis C.

The second locking device is advanced by rotating it with the inner drive portion 102 until the threadless portion 74 extends out of the first locking element 6. Thereby, the second end 72 of the second locking element engages the surface of the rod 100 and presses the rod 100 into the U-shaped recess 54 of the pressure element 5. In Fig. 15, the polyaxial bone screw with the locking device is shown without a tool. The first locking element 6 is screwed into the receiving part and presses onto the free legs 55, 56 of the pressure element 5 to lock the head 3. The second locking element 7 is pressing onto the rod to fix the rod's position in the channel. The rod and the head can be fixed independently from each other, which allows adjustments of the position of the rod while maintaining the angular position of the screw element.

During surgery and before insertion of the locking device, the locking device is taken up by the tool 101. By an operating error with respect to the tool, it may happen that the second locking element is advanced, even if the locking device is not yet inserted into the receiving part. The first and second stop prevent a backing out or loss of the second locking element from the first locking element. Also, when the locking device has already been inserted into the receiving part, subsequent adjustments that require a loosening of the fixation of the rod, can be safely performed because the second locking element abuts against the first stop when it is screwed back by the first drive portion 102. Because the legs 55, 56 of the pressure element 5 extend above the rod 100, when the rod 100 is inserted, the first locking element 6 acts only onto the pressure element 5 and therefore only onto the head 3. The second locking element 7 acts only onto the rod 100.

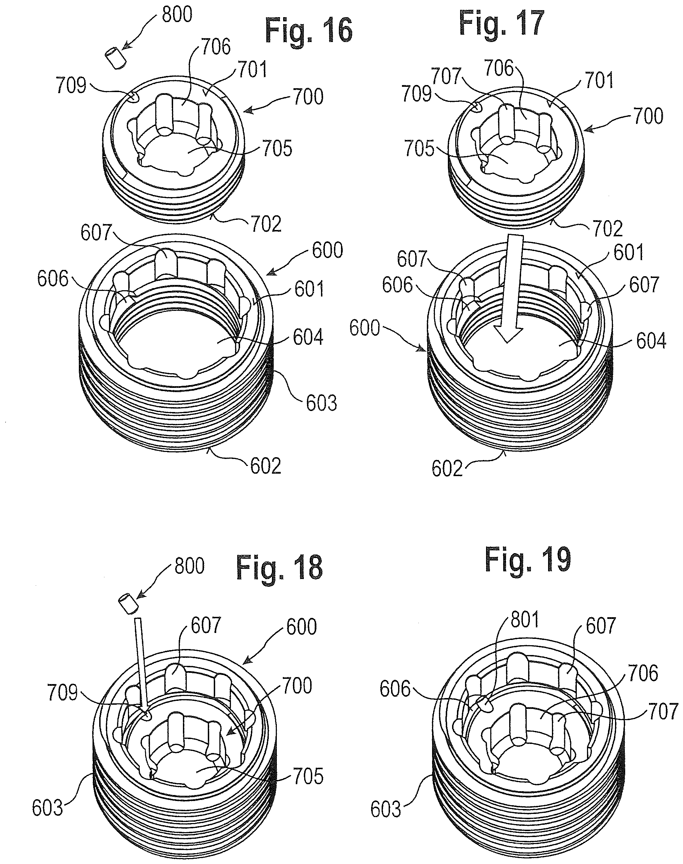

A second embodiment of the locking device is shown in Figs. 16 to 29. It can be used with the polyaxial bone screw as described before or with any other bone anchor. The locking device comprises a first locking element 600 and a second locking element 700. The first locking element 600 and the second locking element 700 are connected with the aid of a pin 800. The pin is an abutment member that provides for a limitation of the advancement of the second locking element 700 in the first locking element 600.

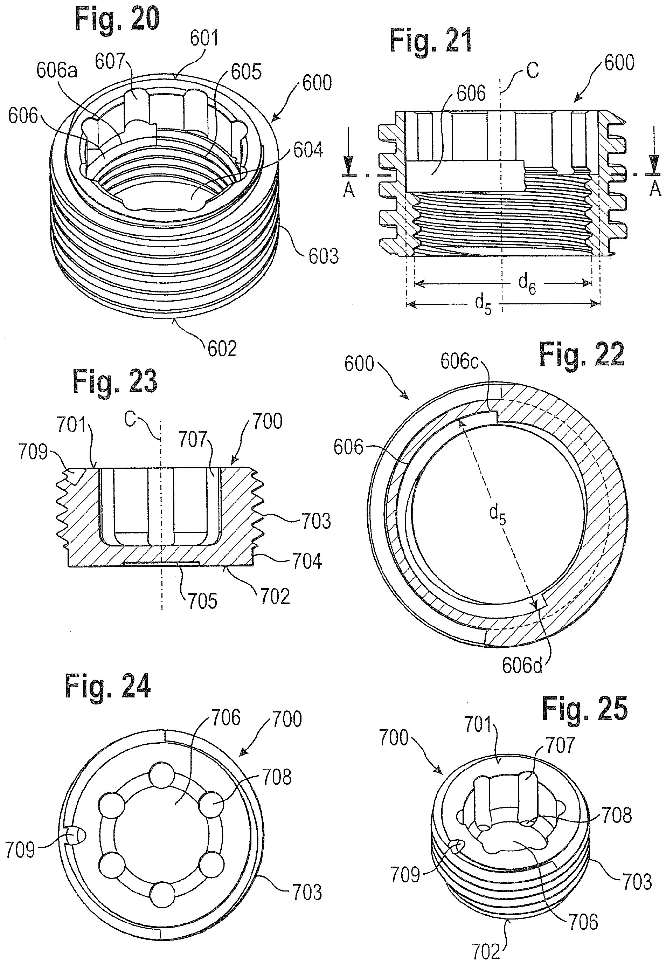

Referring first to Figs. 20 to 22, the first locking element 600 is substantially cylindrical with a first end 601, a second end 602, an external thread portion 603 and a coaxial bore 604. Adjacent the second end an internal thread portion 605 is provided. An engagement structure 607 for engagement with a tool is provided in the region between the internal thread portion 605 and the first end 601. The engagement structure 607 can comprise coaxially extending recesses 607 similar to the first embodiment.

At the transition between the internal thread portion 605 and the portion of the bore 604 that comprises the engagement structure 607, a circumferentially extending groove 606 is provided. The groove 606 extends in the circumferential direction slightly more than half a turn or slightly more than a semi-circle, as can be seen best in Fig. 22. The inner diameter d₅ of the groove is larger than the inner diameter d₆ of the internal thread portion 605 measured between the roots of the internal thread portion 605. The height of the groove 606 in axial direction corresponds to at least one turn of the internal thread portion 605. By the groove a first stop is provided by the one end 606c of the groove in a circumferential direction and a second stop is provided by the other end 606d in a circumferential direction of the groove.

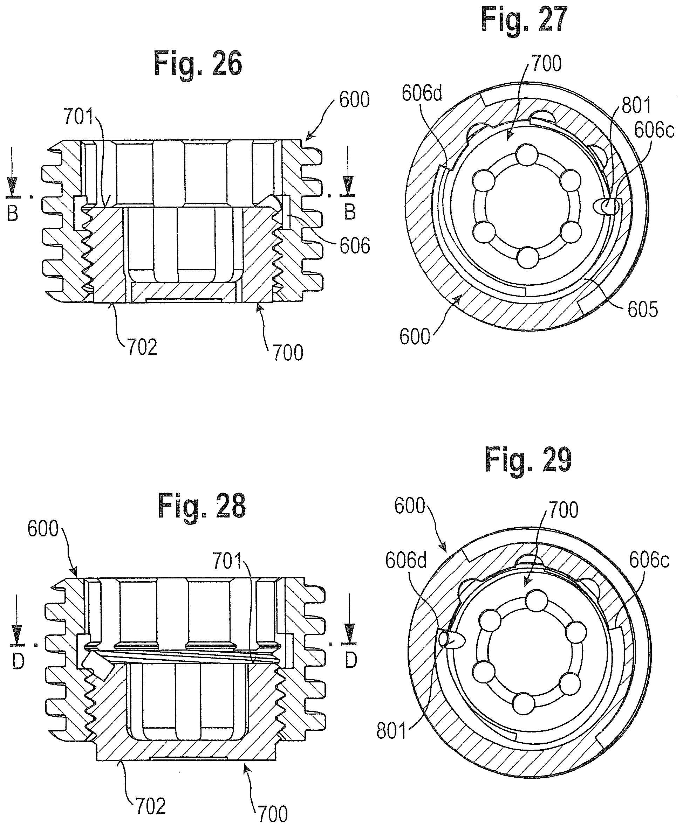

The second locking element 700 has a first end 701, an opposite second end 702 and adjacent the first end 701 an externally threaded portion 703. A coaxial bore, i.e. recess 705 extends from the first end in the direction of the second end. Adjacent the second end 702, a threadless portion 704 is provided. Adjacent the first end 701, a coaxial recess 706 with an engagement structure in the form of, for example, coaxial longitudinal recesses is provided for engagement with a tool. The coaxial engagement recesses 707 extend through the second end 702 thereby providing holes 708. As can be seen in particular in Figs. 23 to 25, a recess 709 is provided for accommodating the pin 800 seen in Figs. 16 to 19. The pin 800 is cylindrical and fits into the recess 709 to such an extent that a portion 801 of the pin projects out of the recess 709. The pin may be fixed to the recess. Furthermore, the pin 800 is sized so as to extend into the groove 606 as shown in Figs. 26 to 29. The pin 800 forms an abutment member that is configured to abut against the first stop formed by the one end 606c of the groove and against the second stop formed by the other end 606d of the groove.

As shown in Figs. 17 to 19, the assembly of the locking device according to the second embodiment is carried out by introducing the second locking element 700 from the first end of the first locking element 600. Once the second locking element 700 is nested into the first locking element 600 by engagement of the threads, the second locking element is advanced towards the second end until the recess 709 is positioned at one of the coaxial recesses 707 of the engagement structure that is at a position in the region of the groove 606. Then, as shown in Fig. 18, the pin 800 is inserted into the recess 709. Thereafter, the second locking element 700 can be rotated within the boundaries defined by the groove 606.

The functioning of the second embodiment of the locking device will be explained with reference to Figs. 26 to 29. In Fig. 26, the second locking element 700 is in the first position.

The outwardly projecting portion 801 of the pin 800 abuts against the one end 606c of the groove 606. The second end 702 of the second locking element 700 does not project out of the first locking element 600. In this preassembled state, the locking device can be delivered and used during surgery.

In Fig. 28, the second locking element 700 has been rotated and advanced downward until the outwardly projecting portion 801 of the pin 800 abuts against the other end 600d of the groove 600. In this position, the second locking element 700 can no longer be advanced downward and therefore is prevented from backing out or escaping out of the first locking element 600. The length of the threadless portion 704 in axial direction is such that in the second position, shown in Fig. 28 the portion that projects outward from the first locking element is suitable for pressing onto the rod and fixing the rod.

As in the first embodiment, the external threaded portion 603 of the first locking element can have any thread form, but preferably has a thread form that prevents splaying of the legs of the receiving part, in particular, a flat thread as shown, or a negative angle thread. The internal thread 605 and the cooperating external thread of the second locking element may have a metric thread form or may also have a flat thread form.

The material of the parts and components of the locking device according to the second embodiment are the same or similar to those of the first embodiment.

The second locking element may be introduced into the first locking element also from the second end. Since the second stop of the locking device is in axial direction approximately in the middle of the first locking element, the locking device according to the second embodiment can be designed more compact.

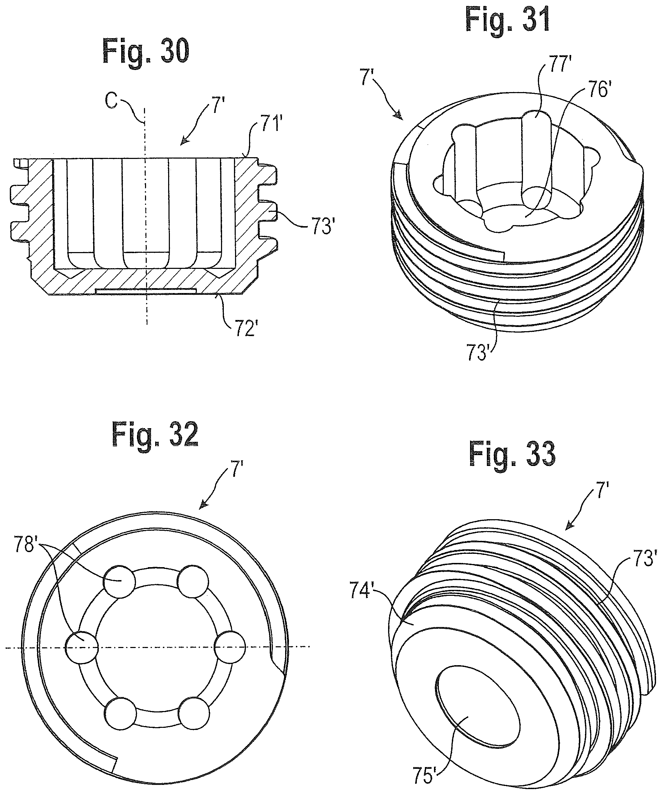

In a modification the first locking element 6' comprises an internal flat thread 63' and the second locking element 7' comprises an external flat thread 73' as can be seen from Figs. 30 to 35b. The thread form is the only difference referring to the first locking element 6 and the second locking element 7 according to the first embodiment.

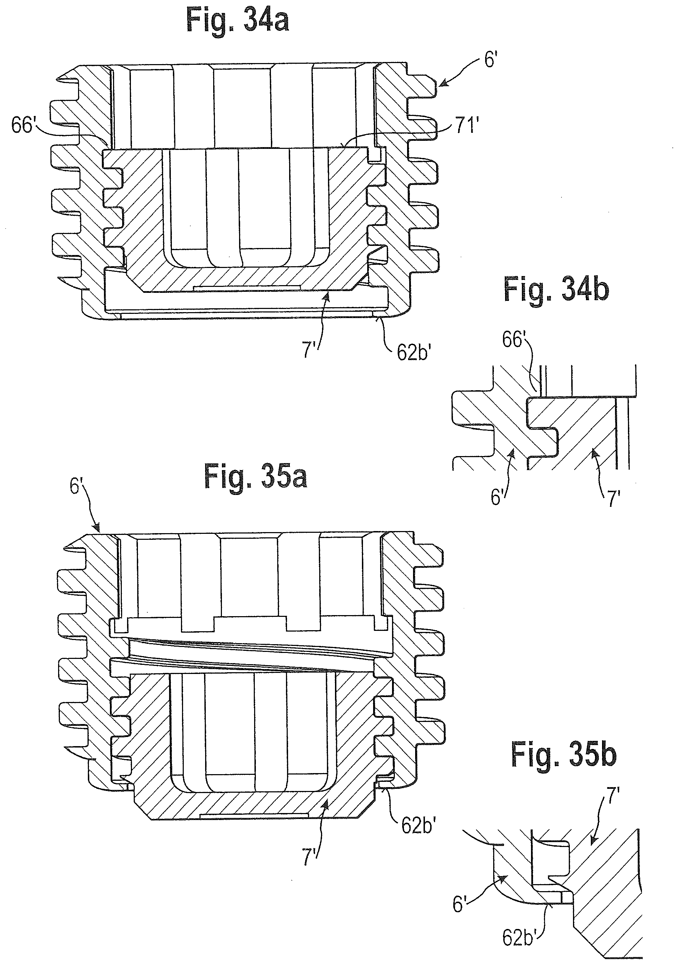

Figs. 34a and 34b show the second locking element 7' abutting against the abutment 66' with its first end 71'.

Fig. 35a and 35b show the second locking element 7' abutting against the annular abutment 62b' with its second end 72'.

Referring to the second embodiment the above mentioned flat thread configuration is also possible.

One advantage of the flat thread configuration referring to the inner thread of the first locking element 6' and the outer thread of the second locking element 7' is that proper end stops are defined because a flat thread is more robust and does not have sharp edges which could violate the integral structure of these end stops. Because of the robust design of the flat thread smaller dimensioning is possible. This effect will be intensified since no radial forces have to be taken up within the flat thread.

Further modifications of the embodiments described are conceivable. A locking device can be used also with a monoaxial screw or with a bone plate. For example, the locking device can be used with a monoaxial screw and a rod that is made from an elastomeric material, which is used for dynamic stabilization.

The second locking element may have at its second end that faces the rod engagement structures such as projections to enhance engagement with the rod.

The engagement structures in the first and second locking element are not limited to the coaxial grooves. They can have any other shape. Alternatively, an engagement structure at the surface of the first end of the first and/or second locking element may be provided.

Other constructions of the stops for limiting the movement of the second locking element in the first locking element are conceivable. For example, the first locking element may have an abutment member that abuts in a groove of the second locking element. The independent head and rod fixation can be realized by other means. For example, the pressure element can be shaped differently without legs that extend above the rod. In this case, the first locking element may have a structure at its second end that presses onto the pressure element.

The polyaxial bone anchor can be realized in many different known manners that are configured to engage with the locking device, for example a polyaxial bone anchor with a favored angle bottom configuration or with a bottom loader configuration.

Particular examples of the locking device and the bone anchoring not falling in the scope of the claims are described below.

A locking device for securing a rod-shaped element in a receiving part of a bone anchor according to a first example, said locking device comprising:
a first locking member 6, 600, 6' having a first end 61, 601 and a second end 62, 602 which is provided with an external thread 63, 603 on at least a portion thereof, a coaxial bore 64, 604 passing entirely through the first locking member the coaxial bore having a bore axis C and an internal thread 65, 605 provided in at least a portion of said bore,
a second locking member 7, 700, 7', the second locking member having a first end 71, 701 and a second end 72, 702, an outer surface with an external thread 73, 703 in at least a portion thereof which cooperates with the internal thread provided at said bore of the first locking member,
wherein the second locking member 7, 700, 7' is movable along the bore axis C by rotating it and wherein a path of advancement of the second locking member is limited towards the first end by a first stop 66, 606c and towards the second end 62b, 606d by a second stop.

The locking device according to a second example corresponding to the locking device of the first aspect, wherein in an assembled state, the first 6, 600 and the second 7, 700 locking member are inseparable.

The locking device according to a third example corresponding to the locking device of the first or second aspect, wherein when the second locking member 7, 700, 7' is in a first position defined by the first stop 66, 606c, its first end 71, 701 facing the first end 61, 601 of the first locking member 6, 600 is within the bore.

The locking device according to a fourth example corresponding to any of the locking devices of the first to the third aspect, wherein when the second locking member 7, 700, 7' is in a second position defined by the second stop, at least a portion of the second locking member 74, 704 protrudes outward of the bore.

The locking device according to a fifth example corresponding to any of the locking devices of the first to the fourth aspect, wherein the first locking element 6, 600 has an engagement structure 67, 607 for engagement with a tool provided at the first end 61, 601 and extending into the bore 64, 604.

The locking device according to a sixth example corresponding to any of the locking devices of the first to the fifth aspect, wherein the first stop 66, 606c is provided at a distance from the first end 61, 601.

The locking device according to a seventh example corresponding to any of the locking devices of the first to the sixth aspect, wherein the first stop 66, 606c is an abutment provided at the first locking element 6, 600, 6'.

The locking device according to an eighth example corresponding to any of the locking devices of the first to the seventh aspect, wherein the first stop is provided by an inner surface portion 66 of the bore 64, the diameter of which is the same as or smaller than the inner diameter between the roots of the internal thread 65 provided at the first locking element.

The locking device according to a ninth example corresponding to any of the locking devices of the first to the eights aspect, wherein the second stop 62b is provided by an abutment at the second end of the first locking element.

The locking device according to a tenth ; example corresponding to any of the locking devices of the first to the ninth aspect, wherein the second stop is a projection 62b projecting into the bore further that the root of the internal thread 65.

The locking device according to an eleventh example corresponding to the locking device of the tenth aspect, wherein the projection 62b is annular.

The locking device according to a twelfth example corresponding to any of the locking devices of the first to the eights aspect, wherein the second stop is provided by an abutment 801, 606 at a distance from the first end and the second end.

The locking device according to a thirteenth example corresponding to the locking device of the twelfth aspect, wherein the first stop is formed by the first end 606c of a groove 606 in the inner wall of the bore that faces the first end 601 of the first locking element 600.

The locking device according to an fourteenth example corresponding to the locking device of the twelfth or thirteenth aspect, wherein the second stop is formed by a second end 606d of a groove 606 in the inner wall of the bore of the first locking element 600 that faces the second end 602.

The locking device according to a fifteenth example corresponding to any of the locking devices of the twelfth to the fourteenth aspect, wherein the second locking element 600 has an abutment member 800, 801 projecting into the groove 606.

The locking device according to a sixteenth example corresponding to any of the locking devices of the twelfth to the fifteenth aspect, wherein the abutment member is a pin 800.

The locking device according to a seventeenth example corresponding to any of the locking devices of the twelfth to the sixteenth aspect, wherein the groove extends circumferentially, preferably about one half turn or more.

A bone anchor according to an eighteenth example comprising an anchoring element 1 comprising a shank 2 to be anchored in a bone or a vertebra; a receiving part 4 connected to the shank 1, the receiving part comprising:
a first end 41 opposite to the shank and a second end 42 facing the shank;
a longitudinal axis C passing through the two ends;
a bore 43 coaxial with the longitudinal axis extending from the first end through at least a portion of the receiving part; and
a substantially U-shaped recess 44 for receiving a rod-shaped element 100, the recess forming two free legs 45, 46 extending in the direction of the first end 41, the legs being provided with an internal thread 47;
and a locking device 67, 600, 700 according to any of the locking devices of the aspects one to seventeen, wherein the external thread 63, 603 of the first locking element is configured to engage the internal thread 47 provided at the legs.

The bone anchor according to a nineteenth example corresponding to the locking device of the eighteenth aspect, wherein said anchoring element comprises a head 3 and wherein said receiving part 4 comprises a region 41 adjacent to said second end for pivotably receiving said head.

The bone anchor according to a twentieth example corresponding to the locking device of the nineteenth aspect, further comprising a pressure element 5 arranged in the receiving part 4 between the head and the locking device for exerting pressure onto said head to lock the head in the receiving part.

The bone anchor according to a twenty-first example corresponding to the locking device of the nineteenth or twentieth aspect, wherein said first locking member 6, 600, 6' is configured to lock the head 3 without fixing the rod-shaped element 100 and wherein said second locking member 7, 700, 7' is configured to fix the rod-shaped element.

## Claims

1. A locking device for securing a rod-shaped element in a receiving part of a bone anchor, said locking device comprising:
a first locking element (6, 600, 6') having a first end (61, 601) and a second end (62, 602) which is provided with an external thread (63, 603) on at least a portion thereof, a coaxial bore (64, 604) passing entirely through the first locking element the coaxial bore having a bore axis (C) and an internal thread (65, 605) provided in at least a portion of said bore,
a second locking element (7, 700, 7'), the second locking element having a first end (71, 701) and a second end (72, 702), an outer surface with an external thread (73, 703) in at least a portion thereof which cooperates with the internal thread provided at said bore of the first locking element, wherein the second locking element (7, 700, 7') is movable along the bore axis (C) by rotating it and wherein a path of advancement of the second locking element is limited towards the first end by a first stop (66, 606c) and towards the second end (62b, 606d) by a second stop,
wherein the first stop (66, 606c) is an abutment provided at the first locking element (6, 600, 6'), and
wherein the external thread (73, 703) of the second locking element (7, 700, 7') and the internal thread (65, 605) of the first locking element (6, 600, 6') are formed as flat threads.

2. The locking device of claim 1, wherein in an assembled state, the first (6, 600) and the second (7, 700) locking elements are inseparable.

3. The locking device of claim 1 or 2, wherein the first stop (66, 606c) is provided at a distance from the first end (61, 601).

4. The locking device of one of claim 1 to 3, wherein the first stop is provided by an inner surface portion (66) of the bore (64), the diameter of which is the same as or smaller than the inner diameter between the crests of the internal thread (65) provided at the first locking element.

5. The locking device of one of claims 1 to 4, wherein the second stop (62b) is provided by an abutment at the second end of the first locking element.

6. The locking device of one of claims 1 to 5, wherein the second stop is a projection (62b) projecting into the bore further that the root of the internal thread (65).

7. The locking device of one of claims 1 to 3, wherein the first stop is formed by the first end (606c) of a groove (606) in the inner wall of the bore that faces the first end (601) of the first locking element (600).

8. The locking device of claim 7, wherein the second stop is formed by a second end (606d) of the groove (606) in the inner wall of the bore of the first locking element (600) that faces the second end (602).

9. The locking device of one of claims 7 or 8, wherein the second locking element (600) has an abutment member (800, 801) projecting into the groove (606).

10. The locking device of claim 9, wherein the abutment member is a pin (800).

11. The locking device of one of claims 7 to 10, wherein the groove extends circumferentially, preferably about one half turn or more.

12. The locking device of one of the preceding claims, wherein the external thread (63, 603) of the first locking element (6, 600, 6') is formed as a flat thread.

13. A bone anchor comprising
an anchoring element (1) comprising a shank (2) to be anchored in a bone or a vertebra;
a receiving part (4) connected to the shank (1), the receiving part comprising:
a first end (41) opposite to the shank and a second end (42) facing the shank;
a longitudinal axis (C) passing through the two ends;
a bore (43) coaxial with the longitudinal axis extending from the first end through at least a portion of the receiving part; and
a substantially U-shaped recess (44) for receiving a rod-shaped element (100), the recess forming two free legs (45, 46) extending in the direction of the first end (41), the legs being provided with an internal thread (47);
and a locking device of one of claims 1 to 12 wherein the external thread (63, 603) of the first locking element is configured to engage the internal thread (47) provided at the legs.

## Patentansprüche

1. Verriegelungsvorrichtung zum Sichern eines stabförmigen Elements in einem Aufnahmeteil eines Knochenankers, wobei die Verriegelungsvorrichtung aufweist:
ein erstes Verriegelungselement (6, 600, 6'), das ein erstes Ende (61, 601) aufweist und ein auf zumindest einem Abschnitt davon mit einem Außengewinde (63, 603) versehenes zweites Ende (62, 602), und eine vollständig durch das erste Verriegelungselement gehende koaxiale Bohrung (64, 604), wobei die koaxiale Bohrung eine Bohrungsachse (C) und ein in zumindest einem Abschnitt der Bohrung vorgesehenes Innengewinde (65, 605) aufweist,
ein zweites Verriegelungselement (7, 700, 7'), wobei das zweite Verriegelungselement ein erstes Ende (71, 701) aufweist und ein zweites Ende (72, 702), und eine äußere Fläche mit einem Außengewinde (73, 703) in zumindest einem Abschnitt davon, das mit dem an der Bohrung des ersten Verriegelungselements vorgesehenen Innengewinde zusammenwirkt,
wobei das zweite Verriegelungselement (7, 700, 7'), indem man es dreht, entlang der Bohrungsachse (C) bewegbar ist, und wobei ein Vorrückweg des zweiten Verriegelungselements zu dem ersten Ende hin durch einen ersten Anschlag (66, 606c) und zu dem zweiten Ende (62b, 606d) hin durch einen zweiten Anschlag begrenzt ist,
wobei der erste Anschlag (66, 606c) ein an dem ersten Verriegelungselement (6, 600, 6') vorgesehenes Widerlager ist, und
wobei das Außengewinde (73, 703) des zweiten Verriegelungselements (7, 700, 7') und das Innengewinde (65, 605) des ersten Verriegelungselements (6, 600, 6') als Flachgewinde ausgebildet sind.

2. Verriegelungsvorrichtung nach Anspruch 1, wobei das erste (6, 600) und das zweite (7, 700) Verriegelungselement in einem zusammengesetzten Zustand untrennbar sind.

3. Verriegelungsvorrichtung nach Anspruch 1 oder 2, wobei der erste Anschlag (66, 606c) in einem Abstand von dem ersten Ende (61, 601) vorgesehen ist.

4. Verriegelungsvorrichtung nach einem der Ansprüche 1 bis 3, wobei der erste Anschlag durch einen inneren Flächenabschnitt (66) der Bohrung (64) gebildet ist, dessen Durchmesser derselbe wie oder kleiner als der innere Durchmesser zwischen den Spitzen des an dem ersten Verriegelungselement vorgesehenen Innengewindes (65) ist.

5. Verriegelungsvorrichtung nach einem der Ansprüche 1 bis 4, wobei der zweite Anschlag (62b) durch ein Widerlager an dem zweiten Ende des ersten Verriegelungselements gebildet ist.

6. Verriegelungsvorrichtung nach einem der Ansprüche 1 bis 5, wobei der zweite Anschlag ein Vorsprung (62b) ist, der weiter als der Gewindegrund des Innengewindes (65) in die Bohrung vorsteht.

7. Verriegelungsvorrichtung nach einem der Ansprüche 1 bis 3, wobei der erste Anschlag durch das erste Ende (606c) einer Nut (606) in der inneren Wand der Bohrung, das dem ersten Ende (601) des ersten Verriegelungselements (600) zugewandt ist, gebildet ist.

8. Verriegelungsvorrichtung nach Anspruch 7, wobei der zweite Anschlag durch ein zweites Ende (606d) der Nut (606) in der inneren Wand der Bohrung des ersten Verriegelungselements (600), das dem zweiten Ende (602) zugewandt ist, gebildet ist.

9. Verriegelungsvorrichtung nach Anspruch 7 oder 8, wobei das zweite Verriegelungselement (600) ein Widerlagerelement (800, 801) aufweist, das in die Nut (606) vorsteht.

10. Verriegelungsvorrichtung nach Anspruch 9, wobei das Widerlagerelement ein Stift (800) ist.

11. Verriegelungsvorrichtung nach einem der Ansprüche 7 bis 10, wobei sich die Nut in eine Umfangsrichtung, vorzugsweise um eine halbe Umdrehung oder mehr, erstreckt.

12. Verriegelungsvorrichtung nach einem der vorangehenden Ansprüche, wobei das Außengewinde (63, 603) des ersten Verriegelungselements (6, 600, 6') als ein Flachgewinde ausgebildet ist.

13. Knochenanker, aufweisend
ein Verankerungselement (1), das einen in einem Knochen oder einem Wirbel zu verankernden Schaft (2) aufweist;
ein mit dem Schaft (1) verbundenes Aufnahmeteil (4), wobei das Aufnahmeteil aufweist:
ein dem Schaft entgegengesetztes erstes Ende (41) und ein dem Schaft zugewandtes zweites Ende (42);
eine durch die zwei Enden gehende Längsachse (C);
eine zu der Längsachse koaxiale Bohrung (43), die sich von dem ersten Ende durch zumindest einen Abschnitt des Aufnahmeteils erstreckt; und
eine im Wesentlichen U-förmige Ausnehmung (44) zum Aufnehmen eines stabförmigen Elements (100), wobei die Ausnehmung zwei freie Schenkel (45, 46) bildet, die sich in der Richtung des ersten Endes (41) erstrecken, wobei die Schenkel ein Innengewinde (47) aufweisen;
und eine Verriegelungsvorrichtung nach einem der Ansprüche 1 bis 12, wobei das Außengewinde (63, 603) des ersten Verriegelungselements ausgebildet ist, mit dem an den Schenkeln vorgesehenen Innengewinde (47) in Eingriff zu gelangen.

## Revendications

1. Dispositif de verrouillage pour fixer un élément en forme de tige dans une partie réceptrice d'un ancrage osseux, ledit dispositif de verrouillage comprenant :
un premier élément de verrouillage (6, 600, 6') comportant une première extrémité (61, 601) et une seconde extrémité (62, 602), un filetage externe (63, 603) étant prévu sur au moins une partie dudit élément de verrouillage, un alésage coaxial (64, 604) traversant entièrement le premier élément de verrouillage, l'alésage coaxial comportant un axe (C) d'alésage et un filetage interne (65, 605) disposé dans au moins une partie dudit alésage,
un second élément de verrouillage (7, 700, 7'), le second élément de verrouillage comportant une première extrémité (71, 701) et une seconde extrémité (72, 702), une surface extérieure avec un filetage externe (73, 703) sur au moins une partie de celle-ci, qui coopère avec le filetage interne disposé au niveau dudit alésage du premier élément de verrouillage,
dans lequel on peut déplacer le second élément de verrouillage (7, 700, 7') le long de l'axe (C) d'alésage en le faisant tourner, et dans lequel un chemin d'avancement du second élément de verrouillage est limité vers la première extrémité par une première butée (66, 606c) et vers la seconde extrémité (62b, 606d) par une seconde butée,
dans lequel la première butée (66, 606c) est un appui disposé au niveau du premier élément de verrouillage (6, 600, 6'), et
dans lequel le filetage externe (73, 703) du second élément de verrouillage (7, 700, 7') et le filetage interne (65, 605) du premier élément de verrouillage (6, 600, 6') sont formés par des filetages plats.

2. Dispositif de verrouillage selon la revendication 1, dans lequel, quand ils sont assemblés, le premier (6, 600) et le second (7, 700) éléments de verrouillage ne peuvent pas être séparés.

3. Dispositif de verrouillage selon la revendication 1 ou 2, dans lequel la première butée (66, 606c) est disposée à une certaine distance de la première extrémité (61, 601).

4. Dispositif de verrouillage selon l'une des revendications 1 à 3, dans lequel la première butée est assurée par une partie (66) de surface intérieure de l'alésage (64), dont le diamètre est égal ou inférieur au diamètre intérieur entre les sommets du filetage interne (65) disposé sur le premier élément de verrouillage.

5. Dispositif de verrouillage selon l'une des revendications 1 à 4, dans lequel la seconde butée (62b) est assurée par un appui au niveau de la seconde extrémité du premier élément de verrouillage.

6. Dispositif de verrouillage selon l'une des revendications 1 à 5, dans lequel la seconde butée est une partie saillante (62b) dépassant dans l'alésage plus loin que le fond du filetage intérieur (65).

7. Dispositif de verrouillage selon l'une des revendications 1 à 3, dans lequel la première butée est constituée par la première extrémité (606c) d'une rainure (606) dans la paroi intérieure de l'alésage qui fait face à la première extrémité (601) du premier élément de verrouillage (600).

8. Dispositif de verrouillage selon la revendication 7, dans lequel la seconde butée est constituée par une seconde extrémité (606d) de la rainure (606) dans la paroi intérieure de l'alésage du premier élément de verrouillage (600) qui fait face à la seconde extrémité (602).

9. Dispositif de verrouillage selon la revendication 7 ou 8, dans lequel le second élément de verrouillage (600) comporte un élément d'appui (800, 801) faisant saillie dans la rainure (606).

10. Dispositif de verrouillage selon la revendication 9, dans lequel l'élément d'appui est une clavette (800).

11. Dispositif de verrouillage selon l'une des revendications 7 à 10, dans lequel la rainure s'étend de manière circonférentielle, de préférence sur environ un demi-tour ou plus.

12. Dispositif de verrouillage selon l'une des revendications précédentes, dans lequel le filetage externe (63, 603) du premier élément de verrouillage (6, 600, 6') est formé par un filetage plat.

13. Ancrage osseux comprenant
un élément d'ancrage (1) comprenant une tige (2) à ancrer dans un os ou une vertèbre ;
une partie réceptrice (4) raccordée à la tige (1), la partie réceptrice comprenant :
une première extrémité (41) du côté opposé à la tige et une seconde extrémité (42) tournée vers la tige ;
un axe longitudinal (C) passant à travers les deux extrémités ;
un alésage (43) coaxial avec l'axe longitudinal s'étendant depuis la première extrémité à travers au moins une partie de la partie réceptrice ; et
un évidement (44) substantiellement en forme de U pour recevoir un élément (100) en forme de tige, l'évidement formant deux pieds (45, 46) libres s'étendant dans la direction de la première extrémité (41), les pieds étant pourvus d'un filetage interne (47) ;
et un dispositif de verrouillage selon l'une des revendications 1 à 12, dans lequel le filetage externe (63, 603) du premier élément de verrouillage est configuré pour coopérer avec le filetage interne (47) disposé au niveau des pieds.
